# FASCICULE DE BREVET EUROPEEN

(11) **EP 0 931 088 B1**
(45) Date de publication et mention de la délivrance du brevet: **09.10.2002**
(21) Numéro de dépôt: 97935607.8
(22) Date de dépôt: 23.07.1997
(51) Int. Cl.: C07H 17/08, A61K 31/70

(54) **NOUVEAUX DERIVES DE L'ERYTHROMYCINE, LEUR PROCEDE DE PREPARATION ET LEUR APPLICATION COMME MEDICAMENTS**
ERYTHROMYCIN-DERIVATE, VERFAHREN ZUR HERSTELLUNG UND VERWENDUNG ALS ARZNEIMITTEL
NOVEL ERYTHROMYCIN DERIVATIVES, METHOD FOR PREPARING SAME AND USE THEREOF AS DRUGS

(30) Priorité: 24.07.1996 FR 9609285
(43) Date de publication de la demande: 28.07.1999
(73) Titulaire: Aventis Pharma S.A., 92160 Antony (FR)
(72) Inventeur: AGOURIDAS, Constantin, F-94130 Nogent-sur-Marne (FR); CHANTOT, Jean-François, F-94130 Nogent-sur-Marne (FR); DENIS, Alexis, F-75011 Paris (FR); PEJAC, Jean-Marie, F-93140 Bondy (FR)
(86) Numéro de dépôt international: FR9701372
(87) Numéro de publication internationale: WO98003530

(56) Documents cités:
- EP-A- 0 487 411
- EP-A- 0 596 802
- EP-A- 0 676 409

## Description

La présente invention concerne de nouveaux dérivés de l'érythromycine, leur procédé de préparation et leur application comme antibiotiques.

L'invention a pour objet les composés de formule (I) : 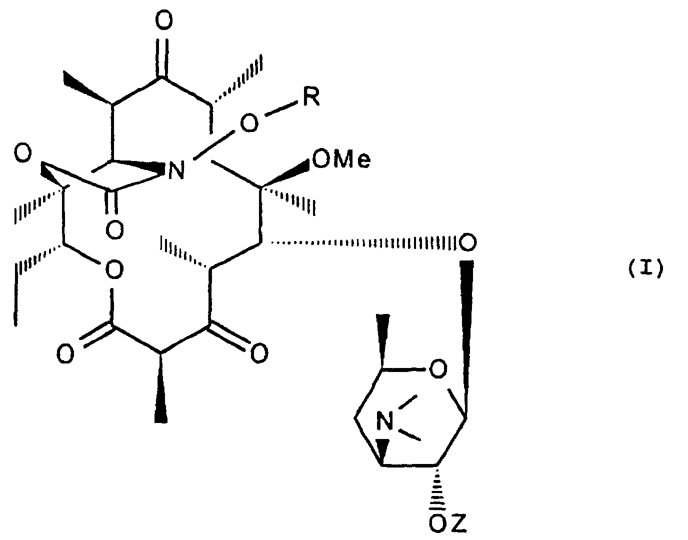 dans lesquels R représente un atome d'hydrogène, un radical alkyle renfermant jusqu'à 12 atomes de carbone, éventuellement substitué par un atome d'halogène ou un radical (CH₂)ₘAr ou un radical 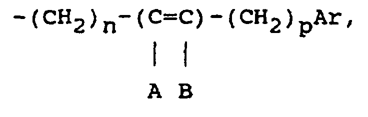 dans lesquels m représente un nombre entier compris entre 1 et 8, n et p identiques ou différents représentent un nombre entier compris entre 0 et 6, A et B identiques ou différents représentent un atome d'hydrogène ou d'halogène ou un radical alkyle renfermant jusqu'à 8 atomes de carbone et Ar représente un radical aryle ou hétéroaryle, éventuellement substitué, et Z représente un atome d'hydrogène ou le reste d'un acide carboxylique renfermant jusqu'à 18 atomes de carbone.

Dans la définition des substituants, le radical alkyle peut être un radical méthyle, éthyle, propyle, isopropyle, n-butyle, isobutyle ou terbutyle, cyclobutyle, cyclopentyle, cyclohexyl, décyle ou dodécyle. L'halogène est de préférence un atome de fluor, de chlore ou de brome. Le radical aryle peut être un radical phényle ou naphtyle. Le radical hétéroaryle peut être un radical thiényle, furyle, pyrolyle, thiazolyle, oxazolyle, imidazolyle, thiadiazolyle, pyrazolyle ou isopyrazolyle, un radical pyridyle, pyrimidyle, pyridazinyle ou pyrazinyle, ou encore un radical indolyle, benzofurannyle, benzothiazyle ou quinoléinyle. Le radical aryle ou hétéroaryle peut être substitué par un ou plusieurs substituants choisis dans le groupe constitué par les radicaux hydroxyle, les atomes d'halogène, les radicaux NO₂, les radicaux C≡N, les radicaux alkyle, alkényle ou alkynyle, O-alkyle, O-alkényle ou O-alkynyle, S-alkyle, S-alkényle ou S-alkynyle et N-alkyle, N-alkényle ou N-alkynyle, renfermant jusqu'à 12 atomes de carbone éventuellement substitués par un ou plusieurs atomes d'halogène, le radical 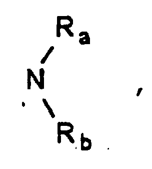 Rₐ et R_{b} identiques ou différents, représentant un atome d'hydrogène ou un radical alkyle renfermant jusqu'à 12 atomes de carbone, le radical 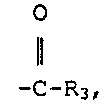 R₃ représentant un radical alkyle renfermant jusqu'à 12 atomes de carbone, ou un radical aryle ou hétéroaryle à 5 ou 6 chaînons comportant un ou plusieurs hétéro-atomes, éventuellement substitués par un ou plusieurs des substituants mentionnés ci-dessus.

Comme radical hétérocycle préféré, on peut citer les radicaux suivants : 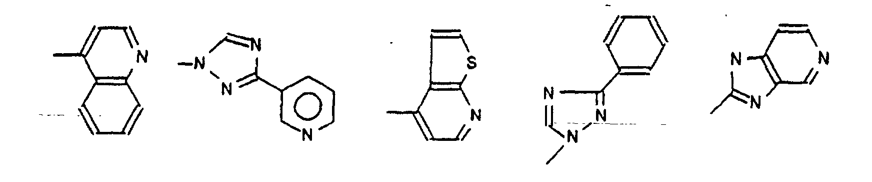 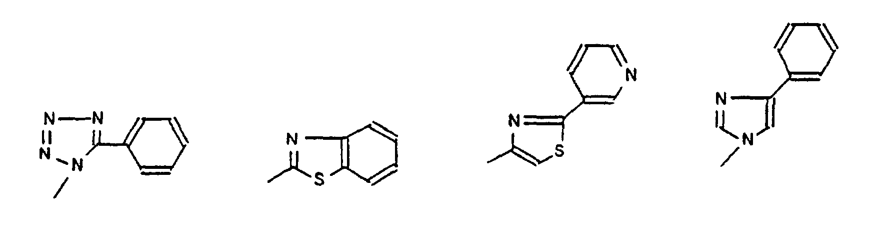 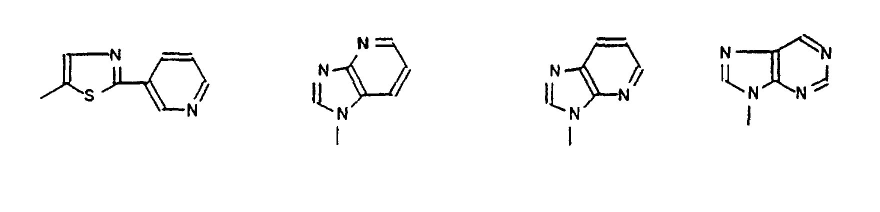 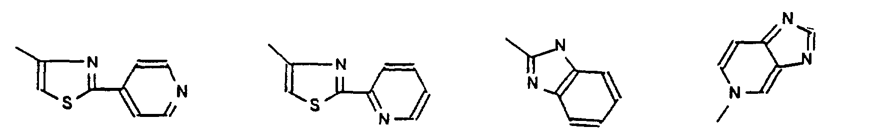 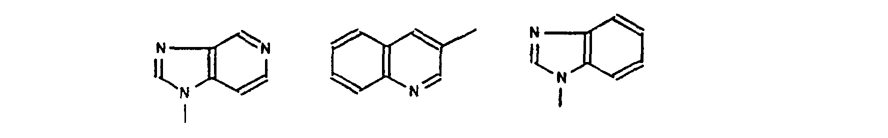 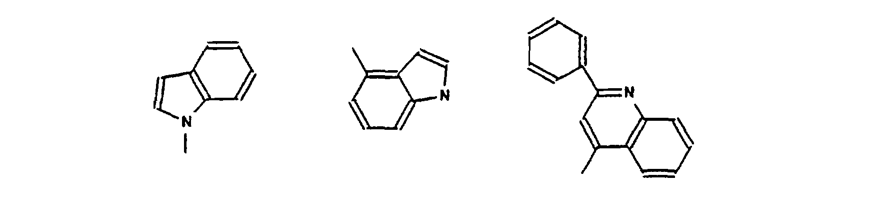 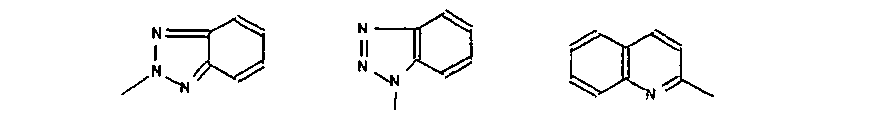 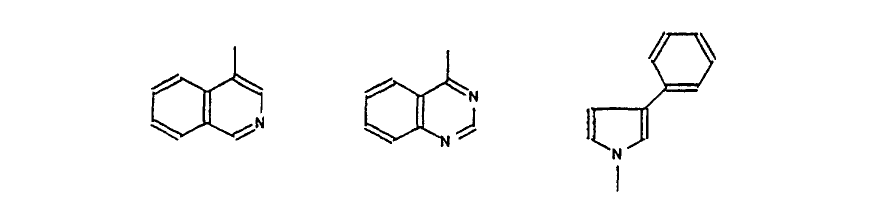 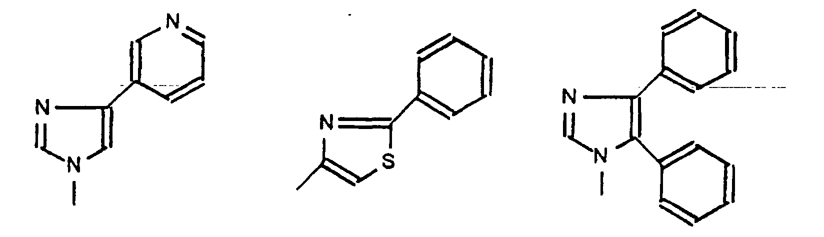 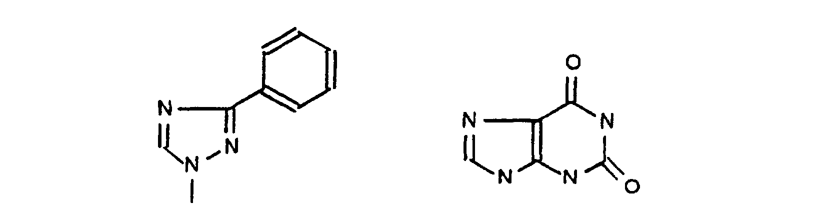 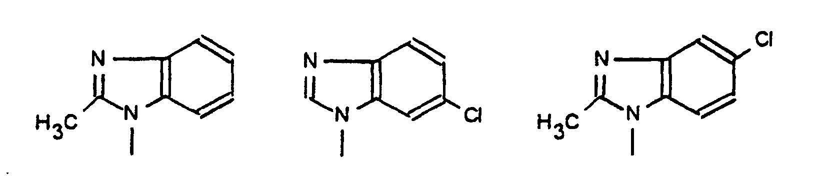 et les radicaux hétérocycliques envisagés dans les demandes de brevets européens 487411, 596802, 676409 et 680967.

Les radicaux peuvent être substitués par un ou plusieurs des substituants indiqués ci-dessus.

L'invention a plus particulièrement pour objet les composés de formule (I) dans lesquels Z représente un atome d'hydrogène, les composés de formule (I) dans lesquels R représente un atome d'hydrogène, les composés de formule (I) dans lesquels R représente (CH₂)ₘAr, m et Ar conservant la même signification que précédemment, et notamment ceux dans lesquels R représente un radical (CH₂)ₘ,Ar dans lequel m' représente le nombre 3, 4, 5 ou 6, comme par exemple ceux dans lesquels Ar représente un radical 4-quinoléinyle éventuellement substitué sur l'un et/ou l'autre des 2 cycles de la quinoléine et tout spécialement ceux dans lesquels Ar représente un radical 4-quinoléinyle non substitué, ou encore les composés de formule (I) dans lesquels Ar représente un radical : 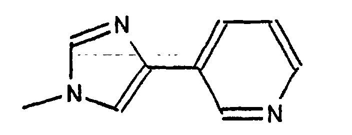 éventuellement substitué sur l'un et/ou l'autre des 2 cycles et tout spécialement ceux dans lesquels le radical Ar n'est pas substitué.

L'invention a tout particulièrement pour objet les composés dont la préparation est donnée ci-après dans la partie expérimentale et notamment le composé de l'exemple 4.

Les produits de formule générale (I) possèdent une très bonne activité antibiotique sur les bactéries gram ^{⊕} telles que les staphylocoques, les streptocoques, les pneumocoques.

Les composés de l'invention peuvent donc être utilisés comme médicaments dans le traitement des infections à germes sensibles et notamment, dans celui des staphylococcies, telles que les septicémies à staphylocoques, staphylococcies malignes de la face ou cutanées, pyodermites, plaies septiques ou suppurantes, furoncles, anthrax, phlegmons, érysipèles et acné, staphylococcies telles que les angines aiguës primitives ou post-grippales, broncho-pneumonies, suppuration pulmonaires, les streptococcies telles que les angines aiguës, les otites, les sinusites, la scarlatine, les pneumococcies telles que les pneumonies, les bronchites ; la brucellose, la diphtérie, la gonococcie.

Les produits de la présente invention sont également actifs contre les infections dues à des germes comme Haemophilus influenzae, Rickettsies, Mycoplasma pneumoniae, Chlamydia, Legionella, Ureaplasma, Toxoplasma, ou à des germes du genre Mycobactérium.

La présente invention a donc également pour objet, à titre de médicaments et, notamment de médicaments antibiotiques, les produits de formule (I) tels que définis ci-dessus, ainsi que leurs sels d'addition avec les acides minéraux ou organiques pharmaceutiquement acceptables.

L'invention a plus particulièrement pour objet, à titre de médicaments et, notamment de médicaments antibiotiques, les produits des exemples.

L'invention a également pour objet les compositions pharmaceutiques renfermant comme principe actif au moins un des médicaments définis ci-dessus.

Ces compositions peuvent être administrées par voie buccale, rectale, parentérale ou par voie locale en application topique sur la peau et les muqueuses, mais la voie d'administration préférée est la voie buccale.

Elles peuvent être solides ou liquides et se présenter sous les formes pharmaceutiques couramment utilisées en médecine humaine, comme par exemple, les comprimés simples ou dragéifiés, les gélules, les granulés, les suppositoires, les préparations injectables, les pommades, les crèmes, les gels ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

ces compositions peuvent également se présenter sous forme d'une poudre destinée à être dissoute extemporanément dans un véhicule approprié, par exemple de l'eau stérile apyrogène.

La dose administrée est variable selon l'affection traitée, le sujet en cause, la voie d'administration et le produit considéré. Elle peut être, par exemple, comprise entre 50 mg et 500 mg par jour par voie orale, chez l'adulte pour le produit de l'exemple 4.

Les composés de formule (II) utilisés comme produits de départ sont décrits et revendiqués dans la demande de brevet européen 0596802.

L'invention a également pour objet un procédé caractérisé en ce que l'on soumet un composé de formule (II) : 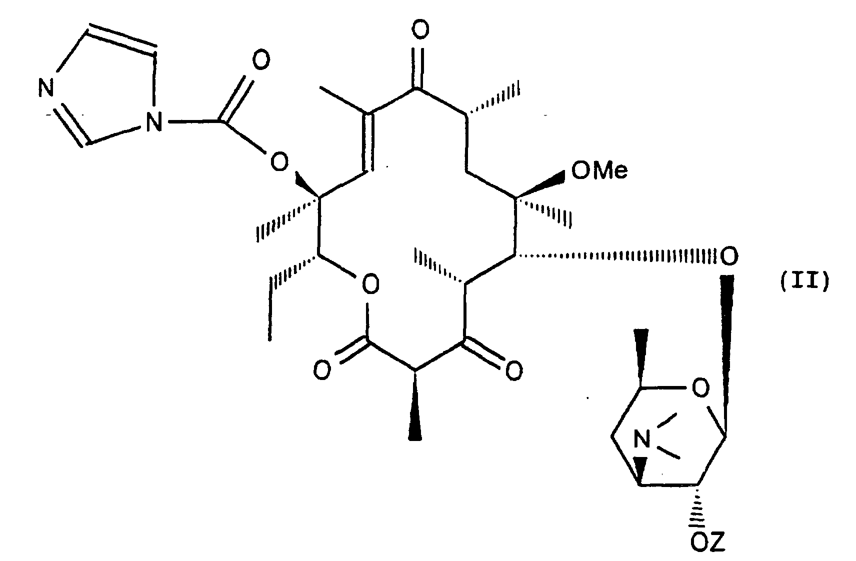 dans lequel Z conserve sa signification précédente, à l'action de l'hydroxylamine ou d'un halogénohydrate d'hydroxylamine, pour obtenir le composé de formule (I_{A}) : 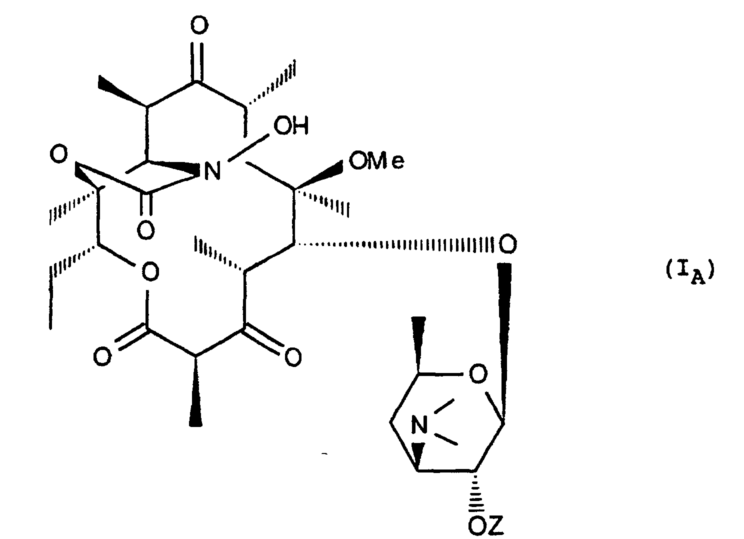 que l'on soumet si désiré à l'action d'un agent de méthanolyse en 2', pour obtenir le composé de formule (I_{B}) correspondant dans lequel Z représente un atome d'hydrogène 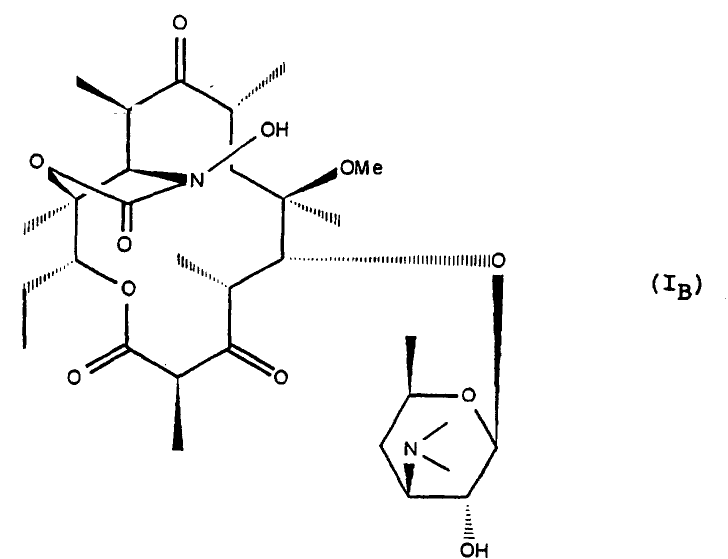 puis soumet le composé (I_{A}) ou (I_{B}) à l'action d'un composé de formule (III) :

R'Hal (III)

dans lequel R' a la même signification que R à l'exception de l'hydrogène et Hal représente un atome d'halogène, pour obtenir le composé de formule (I_{C}) correspondant, que l'on soumet éventuellement à l'action d'un agent d'hydrogénation de la double liaison éventuelle du radical 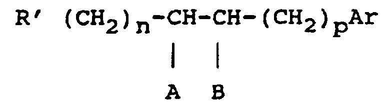 et/ou à l'action d'un agent de libération de la fonction hydroxyle en 2'.

L'invention a également pour objet les composés de formule (III) dont la préparation est donnée ci-après dans la partie expérimentale.

Dans un mode de réalisation préféré du procédé de l'invention :
- on opère en présence d'un excès d'hydroxylamine ou d'halogénohydrate d'hydroxylamine, dans un solvant tel que l'acétonitrile, le dioxane, le diméthylformamide, le tétrahydrofuranne, le diméthoxyéthane ou le diméthylsulfoxyde,
- la libération de l'hydroxyle en 2' est réalisée par méthanolyse,
- l'estérification en 2' est réalisée selon les procédés classiques, Hal dans le composé de formule (III) est de préférence un atome de brome, de chlore ou d'iode,
- la réaction entre le composé (I_{A}) et le composé (III) a lieu en présence d'hydrure de sodium,
- la réduction éventuelle de la chaîne 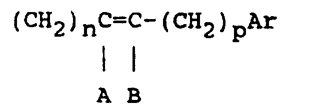 est réalisée à l'aide de l'hydrogène en présence d'un catalyseur tel que le palladium, le platine et indifféremment en présence ou non d'un acide tel que l'acide chlorhydrique ou l'acide acétique.

Les exemples suivants illustrent l'invention :

### EXEMPLE 1 : 11,12-didéoxy-3-de((2,6-didéoxy-3-C-méthyl-3-O-méthyl-alpha-L-ribohexopyranosyl) oxy)-6-O-méthyl-3-oxo-12,11-(oxycarbonyl (hydroxyimino))-érythromycine

On ajoute 200 cm³ d'acétonitrile et 20 g de 2'-acétate de 11-déoxy-10,11-didéhydro 3-de(2,6-didéoxy-3-C-méthyl-3-O-méthyl-alpha-L-ribohexopyranosyl) oxy))-12-O-(11H-imidazol-1-yl) carbonyl)-6-O-méthyl-3-oxo érythromycine dans une solution renfermant 5,9 g de chlorhydrate d'hydroxylamine et 20 cm³ d'eau. On agite pendant 3 heures, évapore à sec, reprend dans le méthanol et agite à nouveau pendant 20 heures à la température ambiante. On évapore à sec, reprend dans le chlorure de méthylène, lave à l'eau, extrait à l'acétate d'éthyle, sèche et évapore à sec. On obtient 17,4 g de produit que l'on chromatographie sur silice en éluant avec le mélange AcOEt-TEA-MeOH (98-0,75-0,75). On obtient 2,06 g de produit par chromatographie en éluant avec le système éther isopropylique-TEA-MeOH (90-5-5). On obtient le produit recherché.
Spectre RMN (400 Mhz dans CDCl₃) ppm
0,88 : CH₃CH₂ ; 1,2 : 8 Me ; 1,25 : 5' Me ; 1,31 : 4 Me ; 1,39 : 2 Me ; 1,33-1,49 : 6 et 12 Me ; 1,55 et 1,94 : CH₂ en 13 ; 1,67 et 1.89 : CH₂ en 7 ; 1,67 : CH₂ en 3' ; 2,27 : N(Me)₂ ; 2,46 : H3' ; 2,68 : 6 OMe ; 2,72 : H8 ; 3,04 : H4 ; 3,14 : H10 ; 3,18 : H2' ; 3,57 : H5' ; 3,82 : H11 ; 3,84 : H2 ; 4,26 : H5 ; 4,35 : H1' ; 5,14 : H13.

### EXEMPLE 2 : (E) 11,12-didéoxy-3-de((2,6-didéoxy-3-C-méthyl-3-O-méthyl-alpha-L-ribohexopyranosyl) oxy)-6-O-méthyl-3-oxo-12,11-(oxycarbonyl (((3-phényl 2-propényl) oxy)imino))-éryt hromycine

On refroidit à 0°C, une solution renfermant 328 mg du produit de l'exemple 1 et 4 cm³ de DMF. On ajoute 41 mg d'hydrure de sodium à 60 % dans l'huile. On agite 15 minutes à 0°C et ajoute 113 mg de bromure de cinnamyle en solution dans 2 ml de DMF sur siliporite. On verse sur la glace, extrait au chlorure de méthylène, lave à l'eau, sèche et évapore à sec. On obtient 420 mg de produit que l'on purifie sur silice en éluant avec le mélange éther isopropylique-triéthylamine-méthanol (9-0,4-0,4). On obtient le produit recherché.
Spectre RMN (300 Mhz dans CDCl₃) ppm
0,74 : CH₃CH₂ ; 1,14 : 8 et 10 Me ; 1,24 : 5' Me ; 1,31 : 4 Me ; 1,41 et 1,49 : 6 et 12 Me ; 1.39 : 2 Me ; 1,50 et 1,82 : CH₂ en 14 ; 1,25 et 1,68 : CH₂ en 4' ; 2,29 : N(Me)₂ ; 2,48 : H3' ; 2,80 : H8 ; 2,83 : 6 Ome ; 3,03 : H10 ; 3,19 : H2' ; 3,54 : H5' ; 3,84 : H2 ; 4,18 : H11 ; 4,24 : H5 ; 4,29 : H1' ; 4,52 et 4,61 : OCH₂CH₃ ; 5,11 : H13 ; 6,40 et 6,76 : éthyléniques E ; 7,2 à 7,43 : aromatiques.

### EXEMPLE 3 : 11,12-didéoxy-3-de((2,6-didéoxy-3-C-méthyl-3-O-méthyl-alpha-L-ribohexopyranosyl) oxy)-6-O-méthyl-3-oxo-12,11-(oxycarbonyl ((3-phénylpropoxy) imimo))-érythromycine

On met sous atmosphère d'hydrogène et agite pendant 2 heures, un mélange renfermant 57 mg du produit de l'exemple 2, 15 ml d'acétate d'éthyle et 8 mg de palladium sur charbon. On filtre, rince à l'acétate d'éthyle et évapore à sec. On obtient 56 mg de produit recherché brut que l'on purifie sur silice en éluant avec le mélange éther isopropylique-triéthylamine-méthanol (9-0,45-0,45). On obtient ainsi 45 mg de produit recherché.
Spectre RMN (CDCl₃) ppm
0,89 : CH₃CH₂ ; 1,13-1,15-1,24-1,29-1,36 : CH₃-CH ; 1,37-1,50 : 6 et 12 Me ; 1,99 : CH₂ central ; 2,27 : N(Me)₂ ; 2,46 : H3' ; 2,69 : 6 OMe ; 2,74 : CH₂Ph et H8 ; 2,97 : H10 ; 3,08 : H4 ; 3,17 : H2' ; 3,52 : H5' ; 3,82 : H2 ; 3,90 et 3,98 : CH₂O-N ; 4,2 : H5 ; 4,21 : H11 ; 4,26 : H1' ; 5,12 : H13 ; 7,1 à 7,3 : Phényl.

### EXEMPLE 4 : 11,12-didéoxy-3-de((2,6-didéoxy-3-C-méthyl-3-O-méthyl-alpha-L-ribohexopyranosyl) oxy)-6-O-méthyl-3-oxo-12,11-(oxycarbonyl (3-(4-quinoléinyl) propoxy) imino)-érythromycine

En opérant comme à l'exemple 2, à partir du produit de l'exemple 1 et du produit de la préparation 1, on a obtenu le produit recherché. F = 224°C.
Spectre RMN (CDCl₃) ppm
0,88 : CH₃CH₂ ; 1,13 : 8 Me ; 1,18 : 10 Me ; 1,23 : 5' Me ; 1,29 : 4 Me ; 1,35 : 2 Me ; 1,34-1,51 : 6 et 12 Me ; 1,25 et 1,69 : CH₂ en 4' ; 1,59 et 1,92 : CH₂ en 7 ; 2,13 : CH₂ central de la chaîne ; 2,28 : N(Me)_{2'} ; 2,45 : H3' ; 2,68 : 6 OMe ; 2,72 : H8 ; 3,05 : H10 ; 3,07 : H4 ; 3,17 : H2' ; 3,28 : CH₂C= ; 3,52 : H5' ; 3,89 : H2 ; 4,01 et 4,08 : CH₂ONC=O ; 4,16 : H11 ; 4,2 : H5 ; 4,27 : H1' ; 5,12 : H13 ; 7,33-7,56-7,69-8,10-8,80 : quinoléine.

### EXEMPLE 5 : 11,12-didéoxy-3-de((2,6-didéoxy-3-C-méthyl-3-O-méthyl-alpha-L-ribohexopyranosyl) oxy)-6-O-méthyl-3-oxo-12,11-(oxycarbonyl (3-(4-(3-pyridinyl)-1H-imidazol-1-yl) propoxy) imino)-érythromycine

En opérant comme à l'exemple 2, à partir du produit de l'exemple 1 et du produit de la préparation 2, on a obtenu le produit recherché.
Spectre RMN (CDCl₃) ppm
0,88 : CH₃CH₂ ; 1,15 : 10 Me ; 1,17 : 8 Me ; 1,24 : 5'Me ; 1,29 : 4 Me ; 1,35 : 2 Me ; 1,37-1,51 : 6 et 12 Me ; 1,30-1,75 : CH₂ en 4' ; 1,62-1,72 : CH₂ en 4' ; 1,62-1,72 : CH₂ en 7 ; 1,60-1,92 : CH₂ en 14 ; 2,17 : CH₂ central ; 2,29 : NMe₂ ; 2,48 : H3' ; 2,68 : 6 O Me et H8 ; 3,06 : H3 ; 3,08 : H10 ; 3,19 : H2' ; 3,53 : H5' ; 2,83 : H2 ; 3,98 : CH₂-N ; 4,05 : H11 ; 4,21 : H5 ; 4,27 : H1' ; 4,15 à 4,40 : NO-CH₂ ; 5,09 : H13 ; 7,49 et 7,68 : imidazole ; 7,29-8,1-8,46-8,90 : pyridine.

### Préparation 1 : 4-(3-iodopropyl) quinoléine

### Stade A : 4-(3-chloropropyl)-2-(diéthoxyphosphinyl)-1(2H)-quinoléinecarboxylate d'éthyle

On refroidit à -65°C une solution de 7,0 g de 2-(dié thoxyphosphinyl) 1(2H)-quinoléinecarboxylate d'éthyle et 70 ml de THF. On ajoute en 15 minutes 17,6 ml de butyllithium et 4 ml de bromochloro-propane. On agite pendant 3 h 30 minutes, verse sur 50 cm³ d'eau glacée, extrait à l'acétate d'éthyle, lave à l'eau, sèche et évapore à sec. On obtient le produit recherché brut que l'on purifie par chromatographie sur silice en éluant avec le mélange cyclohexane-acétate d'éthyle (4-6), on obtient ainsi 6,541 g du produit recherché.
Spectre RMN (250 Mhz dans CDCl₃) ppm
0,98-1,19-1,33 : les CH₃ ; 2,01 : CH₂ central ; 2,55 à 2,85 : =CH₂ ; 3,59 : CH₂-CH₂-X ; 3,71 à 4,14 : CH₂ de POEt ; 4,30 : CH₂ de CO₂Et ; 4,30 : CH₂ de CO₂Et ; 5,57 : P-CH ; 5,92 : =CH-CH-P ; 7,11-7,26-7,60 : Aromatiques.

### Stade B : 4-(3-chloropropyl) quinoléine

On agite à 120°C pendant 2 heures, 6,5 g du produit du stade A, 65 cm³ d'alcool éthylique et 65 cm³ de soude 2N. On évapore, extrait à l'éther, lave à l'eau, sèche et évapore à sec. On obtient 1,87 g de produit que l'on chromatographie sur silice en éluant avec le mélange chlorure de méthylène-acétate d'éthyle (8-2). On obtient 0,617 g du produit recherché.
Spectre RMN (CDCl₃) ppm
2,24 : CH₃ central ; 3,27 : CH₂C= ; 3,62 : CH₂-X ; 7,28-7,59-7,72-8,06-8,13-8,83 : quinoléine.

### Stade C : 4-(3-iodopropyl) quinoléine

On agite pendant 4 heures, à 60°C, un mélange de 268 mg du produit du stade B, 5 ml d'acétone, 1,042 g d'iodure de sodium et 30 mg d'iodure de tétrabutylammonium. On évapore l'acétone, reprend dans le chlorure de méthylène, lave avec une solution de sulfite de sodium à 10 %, sèche et évapore à sec. On obtient 3,91 mg de produit recherché.

### Préparation 2 : 3-[1-(3-bromopropyl)-1H-imidazol-4-yl]-pyridine.

on agite pendant 1 heure à 60°C, une solution renfermant 400 mg de 3-(1H-imidazol-4-yl) pyridine, 3 ml de DMF et 132 mg d'hydrure de sodium à 60 % dans l'huile. On ajoute goutte à goutte une solution de 3,22 g de dibromopropane dans 2 ml de DMF. On agite 1 heure à la température ambiante. On verse sur de l'eau glacée, extrait à l'acétate d'éthyle, sèche et évapore à sec. On chromatographie sur silice le produit obtenu en éluant avec le mélange chlorure de méthylène-méthanol (9-1). On obtient 306 mg de produit recherché.

### EXEMPLE DE COMPOSITION PHARMACEUTIQUE

On a préparé des composés renfermant :

| | |
|---|---|
| Produit de l'exemple 4 | 150 mg |
| Excipient q.s.p. | 1 g |

Détail de l'excipient : amidon, talc, stéarate de magnésium

### ETUDE PHARMACOLOGIOUE DES PRODUITS DE L'INVENTION

### Méthode des dilutions en milieu liquide

On a préparé une série de tubes dans lesquels on répartit une même quantité de milieu nutritif stérile. On distribue dans chaque tube des quantités croissantes du produit à étudier, puis chaque tube est ensemencé avec une souche bactérienne.

Après incubation de vingt-quatre heures à l'étuve à 37°C, l'inhibition de la croissance est appréciée par trans-illumination, ce qui permet de déterminer les concentrations minimales inhibitrices (C.M.I.) exprimées en microgrammes/cm³.

Les résultats suivants ont été obtenus avec le produit de l'exemple 4 (lecture après 24 heures) :

| Souches bactériennes à GRAM+ | |
|---|---|
| Staphylococcus aureus 011UC4 | 0,04 |
| Staphylococcus aureus 011G025I | 0,15 |
| Staphylococcus epidermidis 012G011I | 0,15 |
| Streptococcus pyogenes | ≤ 0,02 |
| groupe A 02A1UC1 | |
| Streptococcus agalactiae | ≤ 0,02 |
| groupe B 02B1HT1 | |
| Streptococcus faecalis | ≤ 0,02 |
| groupe D 02D2UC1 | |
| Streptococcus faecium | ≤ 0,02 |
| groupe D 02D3HT1 | |
| Streptococcus sp | ≤ 0,02 |
| groupe G 02G0GR5 | |
| Streptococcus mitis 02mitCB1 | 0,04 |
| Streptococcus agalactiae | 0,08 |
| groupe B 02B1SJ1 | |
| Streptococcus pneumoniae 032UC1 | ≤ 0,02 |
| Streptococcus pneumoniae 030SJ5 | 0,04 |

De plus, le produit de l'exemple 4 a manifesté une activité intéressante sur les souches bactériennes à gram^{⊖} : Haemophilus Influenzae 351HT3, 351CB12, 351CA1 et 351GR6.

## Revendications

1. Les composés de formule (I) : 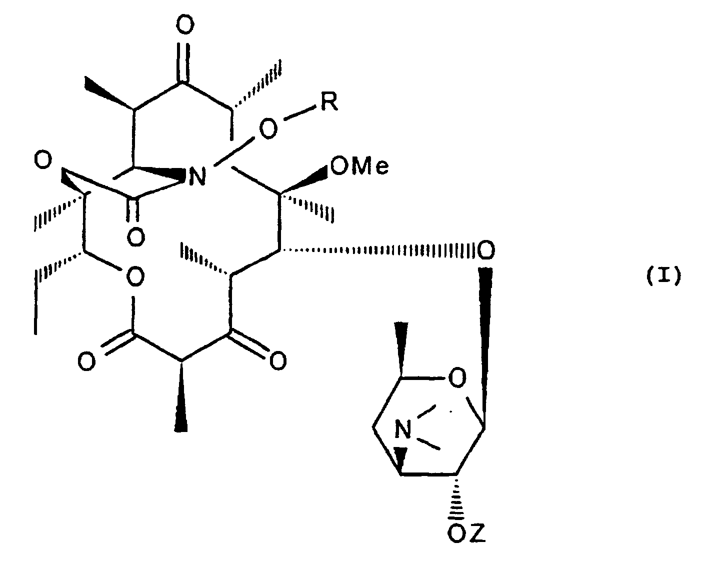 dans lesquels R représente un atome d'hydrogène, un radical alkyle renfermant jusqu'à 12 atomes de carbone, éventuellement substitué par un atome d'halogène ou un radical (CH₂)ₘAr ou un radical 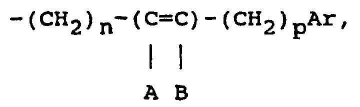 dans lesquels m représente un nombre entier compris entre 1 et 8, n et p identiques ou différents représentent un nombre entier compris entre 0 et 6, A et B identiques ou différents, représentent un atome d'hydrogène ou d'halogène ou un radical alkyle renfermant jusqu'à 8 atomes de carbone et Ar représente un radical aryle ou hétéroaryle, éventuellement substitué, et Z représente un atome d'hydrogène ou le reste d'un acide carboxylique renfermant jusqu'à 18 atomes de carbone.

2. Les composés de formule (I) tels que définis à la revendication 1, dans lesquels Z représente un atome d'hydrogène.

3. Les composés de formule (I) définis à la revendication 1 ou 2, dans lesquels R représente un atome d'hydrogène.

4. Les composés de formule (I) définis à la revendication 1, 2 ou 3 dans lesquels R représente (CH₂)ₘAr, m et Ar conservant la même signification que dans la revendication 1.

5. Les composés de formule (I) définis à la revendication 4, dans lesquels R représente un radical (CH₂)ₘ,Ar dans lequel m' représente le nombre 3, 4, 5 ou 6.

6. Les composés de formule (I) tels que définis à la revendication 5, dans lesquels Ar représente un radical 4-quinoléinyle éventuellement substitué sur l'un et/ou l'autre des 2 cycles de la quinoléine.

7. Les composés de formule (I) tels que définis à la revendication 6, dans lesquels Ar représente un radical 4-quinoléinyle non substitué.

8. Les composés de formule (I) tels que définis à la revendication 5, dans lesquels Ar représente un radical : 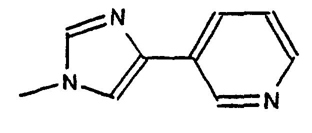 éventuellement substitué sur l'un et/ou l'autre des 2 cycles.

9. Les composés de formule (I) tels que définis à la revendication 8, dans lesquels le radical Ar n'est pas substitué.

10. Les composés de formule (I) définis à la revendication 1 dont le nom suit :
- 11,12-didéoxy-3-de(2,6-didéoxy-3-C-méthyl-3-O-méthyl-alpha-L-ribohexopyranosyl) oxy)-6-O-méthyle-3-oxo-12,11-(oxycarbonyl(3-(4-quinoléinyl) propoxy) imino) érythromycine.

11. A titre de médicaments, les composés de formule (I) définis à l'une quelconque des revendications 1 à 10.

12. Les compositions pharmaceutiques renfermant comme principe actif au moins un médicament selon la revendication 11.

13. Procédé de préparation des composés de formule (I), tels que définis à l'une quelconque des revendications 1 à 10, **caractérisé en ce que** l'on soumet un composé de formule (II) : 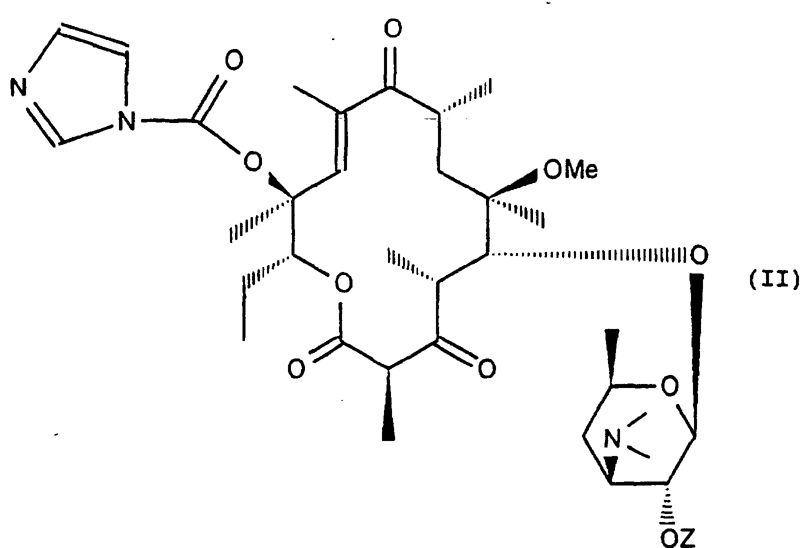 dans lequel Z conserve sa signification précédente, à l'action de l'hydroxylamine ou d'un halogénohydrate d'hydroxylamine, pour obtenir le composé de formule (I_{A}) : 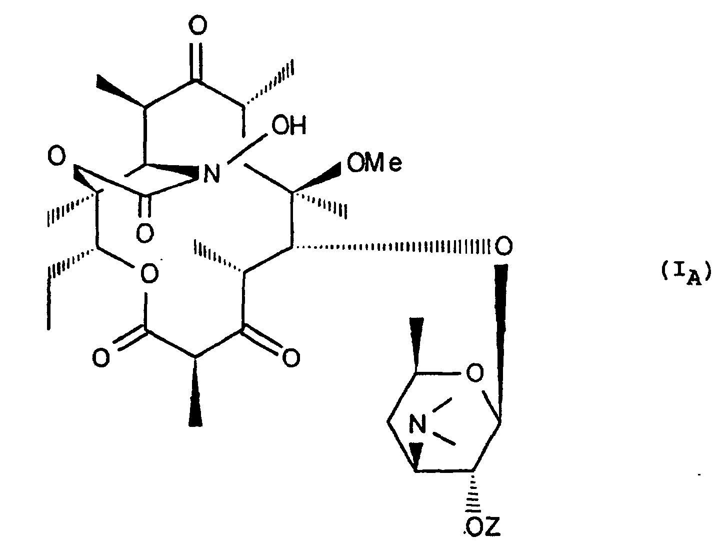 que l'on soumet si désiré à l'action d'un agent de méthanolyse de l'hydroxyle en 2', pour obtenir le composé de formule (I_{B}) correspondant dans lequel Z représente un atome d'hydrogène 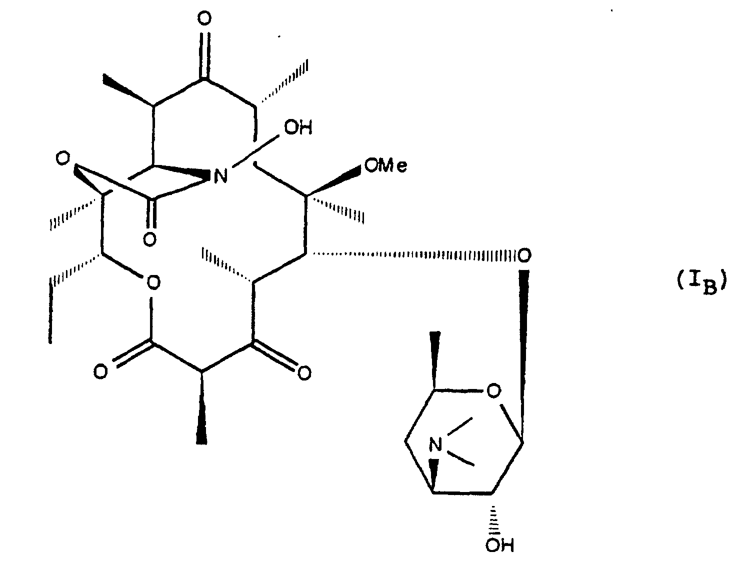 puis soumet le composé (I_{A}) ou (I_{B}) à l'action d'un composé de formule (III) :
R'Hal (III)
dans lequel R' a la même signification que R à l'exception de l'hydrogène et Hal représente un atome d'halogène, pour obtenir le composé de formule (I_{C}) correspondant, que l'on soumet éventuellement à l'action d'un agent d'hydrogénation de la double liaison éventuelle du radical 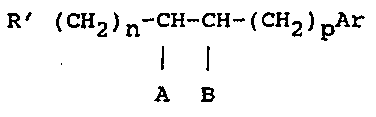 et/ou à l'action d'un agent de libération de la fonction hydroxyle en 2'.

14. A titre de produits chimiques nouveaux, les composés de formule (III) dont les noms suivent :
- 4-(3-iodopropyl)-quinoléine,
- 3-[1-(3-bromopropyl)-1H-imidazol-4-yl]-pyridine.

## Claims

1. The compounds of formula (I) : 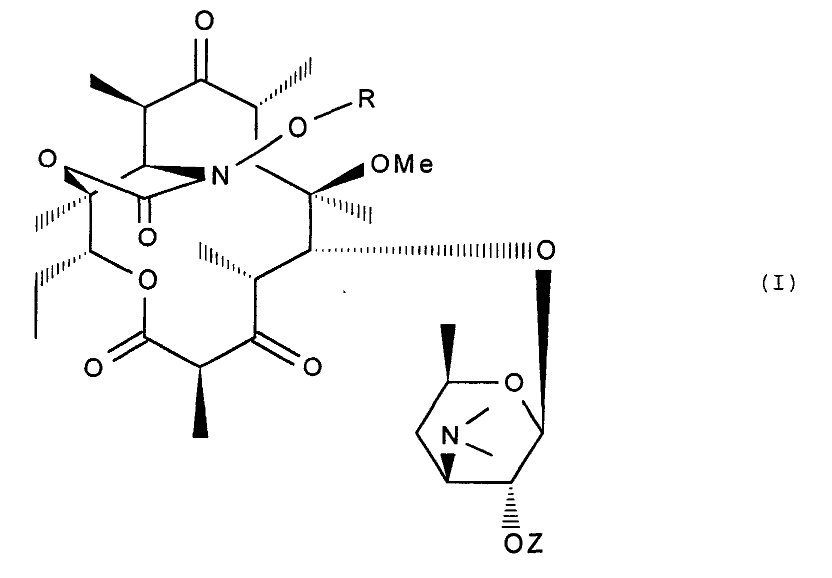 in which R represents a hydrogen atom, an alkyl radical containing up to 12 carbon atoms, optionally substituted by a halogen atom or a (CH₂)ₘAr radical or a 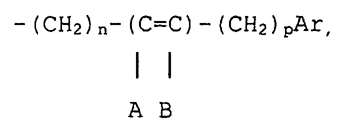 radical, in which m represents an integer between 1 and 8, n and p identical or different represent an integer between 0 and 6, A and B identical or different, represent a hydrogen or halogen atom or an alkyl radical containing up to 8 carbon atoms and Ar represents an optionally substituted aryl or heteroaryl radical, and Z represents a hydrogen atom or the remainder of a carboxylic acid containing up to 18 carbon atoms.

2. The compounds of formula (I) as defined in claim 1, in which Z represents a hydrogen atom.

3. The compounds of formula (I) defined in claim 1 or 2, in which R represents a hydrogen atom.

4. The compounds of formula (I) defined in claim 1, 2 or 3 in which R represents (CH₂)ₘAr, m and Ar retaining the same meaning as in claim 1.

5. The compounds of formula (I) defined in claim 4, in which R represents a (CH₂)ₘ/Ar radical in which m' represents the number 3, 4, 5 or 6.

6. The compounds of formula (I) as defined in claim 5, in which Ar represents a 4-quinolinyl radical optionally substituted on one and/or the other of the 2 quinoline rings.

7. The compounds of formula (I) as defined in claim 6, in which Ar represents a non-substituted 4-quinolinyl radical.

8. The compounds of formula (I) as defined in claim 5, in which Ar represents an: 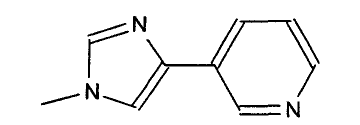 radical optionally substituted on one and/or the other of the 2 rings.

9. The compounds of formula (I) as defined in claim 8, in which the Ar radical is not substituted.

10. The compounds of formula (I) defined in claim 1 the name of which follows:
- 11,12-dideoxy-3-de(2,6-dideoxy-3-C-methyl-3-O-methyl-alpha-L-ribohexopyranosyl) oxy)-6-O-methyl-3-oxo-12,11-(oxycarbonyl(3-(4-quinolinyl) propoxy) imino) erythromycin.

11. As medicaments, the compounds of formula (I) defined in any one of claims 1 to 10.

12. Pharmaceutical compositions containing as active ingredient at least one medicament according to claim 11.

13. Process for the preparation of the compounds of formula (I), as defined in any one of claims 1 to 10, **characterized in that** a compound of formula (II): 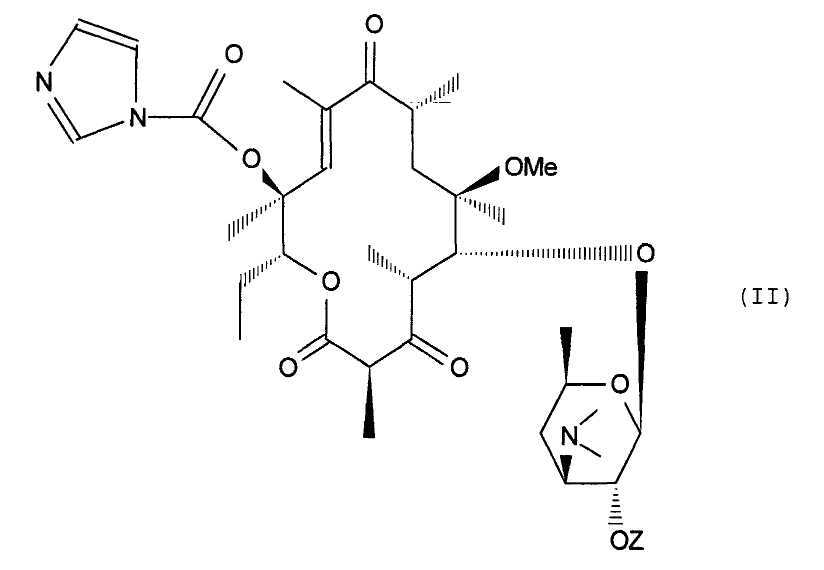 in which Z retains its previous meaning, is subjected to the action of hydroxylamine or a hydroxylamine hydrohalide, in order to obtain the compound of formula (I_{A}): 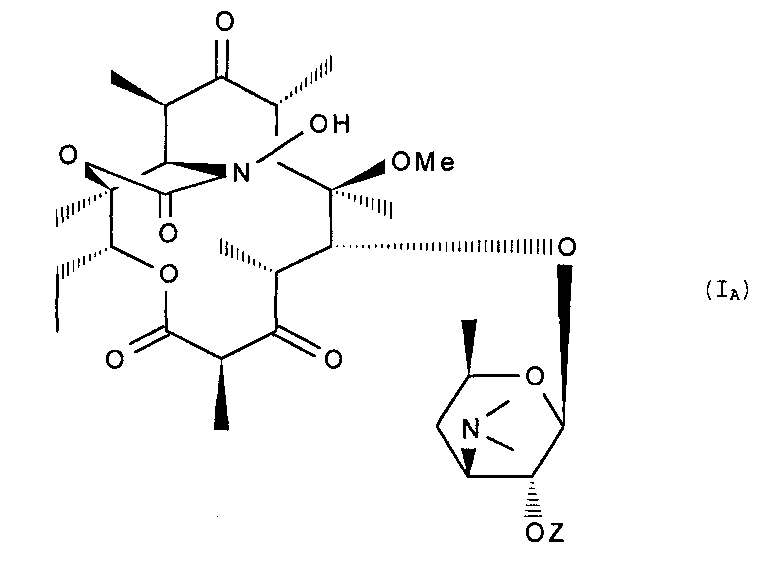 which is subjected if desired to the action of a methanolysis agent of the hydroxyl in position 2', in order to obtain the corresponding compound of formula (I_{B}) in which Z represents a hydrogen atom 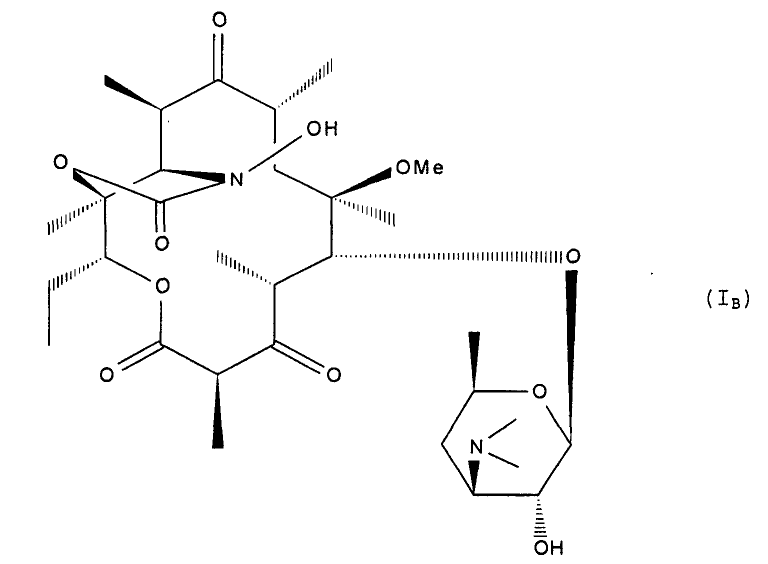 then compound (I_{A}) or (I_{B}) is subjected to the action of a compound of formula (III):
R'Hal (III)
in which R' has the same meaning as R with the exception of hydrogen and Hal represents a halogen atom, in order to obtain the corresponding compound of formula (I_{C}), which is optionally subjected to the action of a hydrogenation agent of the optional double bond of the 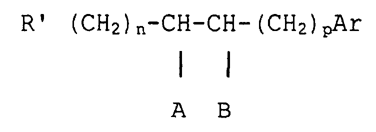 radical and/or to the action of an agent which releases the hydroxyl function in position 2'.

14. As new chemical products, the compounds of formula (III) the names of which follow:
- 4-(3-iodopropyl)-quinoline,
- 3-[1-(3-bromopropyl)-1H-imidazol-4-yl]-pyridine.

## Patentansprüche

1. Verbindungen der Formel (I) 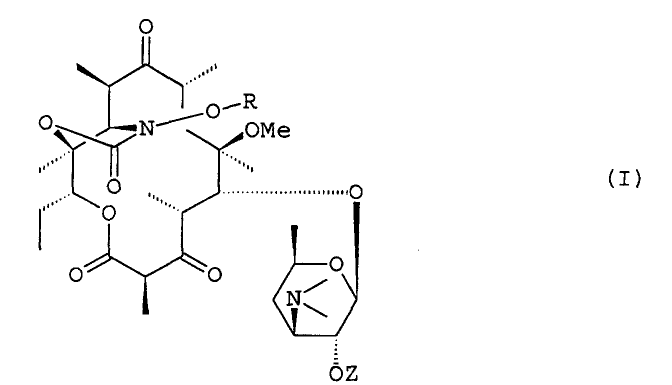 in der
R ein Wasserstoffatom, einen Rest Alkyl mit bis zu 12 Kohlenstoffatomen darstellt, gegebenenfalls substituiert durch ein Halogenatom oder durch einen Rest (CH₂)ₘAr oder durch einen Rest 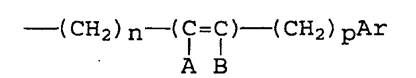 worin m eine ganze Zahl zwischen 1 und 8 bedeutet, n und p, gleich oder verschieden, eine ganze Zahl zwischen 0 und 6 darstellen, A und B, gleich oder verschieden, ein Wasserstoffatom oder ein Halogenatom oder einen Rest Alkyl mit bis zu 8 Kohlenstoffatomen bedeuten und Ar ein Rest Aryl oder Heteroaryl ist, gegebenenfalls substituiert, und Z ein Wasserstoffatom oder den Rest einer Carbonsäure mit bis zu 18 Kohlenstoffatomen darstellt.

2. Verbindungen der Formel (I) wie in Anspruch 1 definiert, worin Z ein Wasserstoffatom darstellt.

3. Verbindungen der Formel (I) wie in Anspruch 1 oder 2 definiert, worin R ein Wasserstoffatom darstellt.

4. Verbindungen der Formel (I) wie in Anspruch 1, 2 oder 3 definiert, worin R (CH₂)ₘAr darstellt, wobei m und Ar die gleiche Bedeutung wie in Anspruch 1 beibehalten.

5. Verbindungen der Formel (I) wie in Anspruch 4 definiert, worin R einen Rest (CH₂)ₘ.Ar darstellt, wobei m' die Zahl 3, 4, 5 oder 6 bedeutet.

6. Verbindungen der Formel (I) wie in Anspruch 5 definiert, worin Ar einen Rest 4-Chinolinyl darstellt, gegebenenfalls substituiert an dem einen und/oder anderen der beiden Chinolin-Ringe.

7. Verbindungen der Formel (I) wie in Anspruch 6 definiert, worin Ar einen nicht substituierten Rest 4-Chinolinyl darstellt.

8. Verbindungen der Formel (I) wie in Anspruch 5 definiert, worin Ar einen Rest 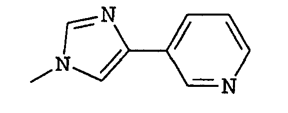 darstellt, gegebenenfalls substituiert an dem einen und/oder anderen der beiden Ringe.

9. Verbindungen der Formel (I) wie in Anspruch 8 definiert, worin der Rest Ar nicht substituiert ist.

10. Verbindungen der Formel (I) wie in Anspruch 1 definiert, mit den folgenden Namen:
- 11,12-Dideoxy-3-de-[(2,6-dideoxy-3-C-methyl-3-O-methyl-alpha-Lribohexopyranosyl)-oxy]-6-O-methyl-3-oxo-12,11-{oxycarbonyl-[3-(4-chinolinyl)-propoxy]-imino}-erythromycin.

11. Als Arzneimittel die Verbindungen der Formel (I) wie in irgendeinem der Ansprüche 1 bis 10 definiert.

12. Pharmazeutische Zusammensetzungen, enthaltend als Wirkstoff mindestens ein Arzneimittel nach Anspruch 11.

13. Verfahren zur Herstellung der Verbindungen der Formel (I) wie in irgendeinem der Ansprüche 1 bis 10 definiert, **dadurch gekennzeichnet, daß** man eine Verbindung der Formel (II) 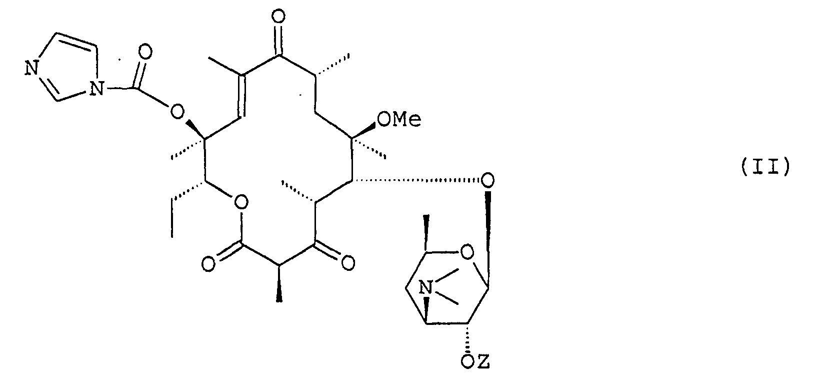 in der Z seine vorstehende Bedeutung beibehält, der Einwirkung von Hydroxylamin oder eines Hydroxylammonium-Halogenids unterzieht, um die Verbindung der Formel (I_{A}) 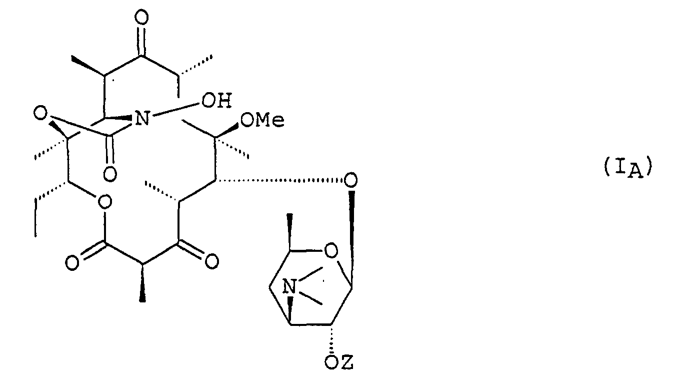 zu erhalten, die man dann, wenn erwünscht, der Einwirkung eines Mittels zur Methanolyse des Hydroxyls in 2' unterzieht, um die entsprechende Verbindung der Formel (I_{B}) zu erhalten, in der Z ein Wasserstoffatom ist: 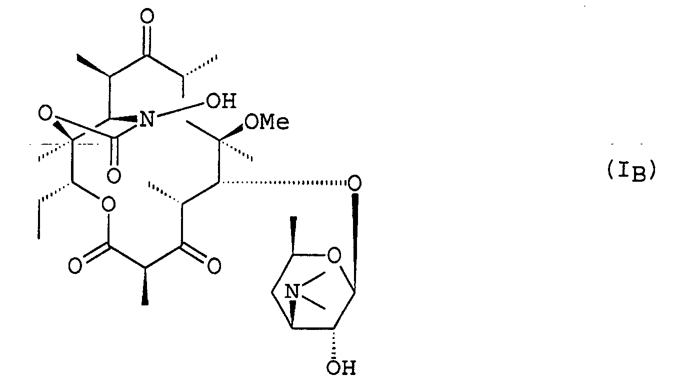 und man anschließend die Verbindung (I_{A}) oder (I_{B}) der Einwirkung einer Verbindung der Formel (III)
R'Hal (III)
unterzieht, in der R' die gleiche Bedeutung wie R besitzt, mit der Ausnahme von Wasserstoff, und Hal ein Halogenatom darstellt, um die entsprechende Verbindung der Formel (I_{C}) zu erhalten, die man gegebenenfalls der Einwirkung eines Mittels zur Hydrierung der eventuellen Doppelbindung des Restes 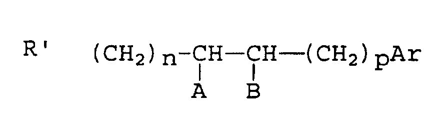 und/oder der Einwirkung eines Mittels zur Freisetzung der Hydroxyl-Funktion in 2' unterzieht.

14. Als neue chemische Produkte die Verbindungen der Formel (III) mit den folgenden Namen:
- 4-(3-Iodpropyl)-chinolin,
- 3-[1-(3-Brompropyl)-1H-imidazol-4-yl]-pyridin.
